# EUROPEAN PATENT APPLICATION

(11) **EP 4 241 801 A1**
(43) Date of publication of application: **13.09.2023**
(21) Application number: 23160889.4
(22) Date of filing: 09.03.2023
(51) Int. Cl.: A61M 1/02

(54) **OPTIMIZATION OF FLUID POOLING**

(30) Priority: 11.03.2022 US 202263319066 P
(71) Applicant: Fenwal, Inc., Lake Zurich, IL 60047 (US)
(72) Inventor: KUSTERS, Benjamin E., Lake Zurich, 60047 (US); MIN, Kyungyoon, Lake Zurich, 60047 (US)
(74) Representative: Maikowski & Ninnemann Patentanwälte Partnerschaft mbB

(57) **Abstract**

A method (and a system for implementing such method) for creating a plurality of fluid products each having a minimum content of a fluid component includes providing a plurality of intermediate fluid volumes each having a known content of a fluid component. The intermediate fluid volumes are grouped by fluid component content, followed by a determination of whether two of the intermediate fluid volumes may be pooled to achieve the minimum content for a final fluid product. The method proceeds with creating combinations of three and then more intermediate fluid volumes achieving the minimum content for a final fluid product, followed by creating combinations of intermediate fluid volumes exceeding the minimum content. Each intermediate fluid volume is assigned to only one of the combinations, with the intermediate fluid volumes being assigned to the combinations so as to maximize the number of combinations and, thus, the number of final fluid products.

## Description

### Cross-Reference To Related Applications

This application claims the benefit of and priority of U.S. Provisional Patent Application Serial No. 63/319,066, filed March 11, 2022, the contents of which are incorporated by reference herein.

### Field of the Disclosure

The present disclosure relates to fluid pooling. More particularly, the present disclosure relates to pooling a plurality of intermediate fluid volumes to create fluid products having a target content of a fluid component.

### Description of Related Art

Whole blood collected from donors is often separated into components that are extremely valuable for medical therapies. Often these components are not present in a therapeutic dose, or the amount required for a procedure such as a transfusion. A number of source bags containing the desired component are then combined or "pooled" into a single bag to accomplish this therapeutic dose.

Common desired blood components include platelets which can be found in various platelet products such as buffy coat, platelet rich plasma, or platelet concentrate. There are several methods to pool platelet products in order to achieve a desirable therapeutic dose of platelets, such as 3.0e11 platelets.

This is currently accomplished using buffy coat or platelet rich plasma methods. The buffy coat method involves separating units of whole blood into red blood cells, platelet poor plasma, and buffy coat. The buffy coat content derived from multiple whole blood units are pooled and processed via a second separation to produce a platelet concentrate that forms the therapeutic platelet product. The platelet rich plasma method is similar, but rather than producing a buffy coat in the first separation, the whole blood is separated into red blood cells and platelet rich plasma. The platelet rich plasma content from multiple whole blood units is then pooled and processed via a second separation to produce the platelet product. In both methods, the platelet content of the intermediate component (buffy coat or platelet rich plasma) is unknown, and thus the process must pool enough units, typically four, to ensure the platelet content of the final product will achieve dosing requirements, such as 3.0e11 platelets. Therefore, the current process may lead to inefficiencies that prevent production of a maximum number of products from a specific number of whole blood units.

It is therefore desirable to utilize a more efficient pooling method that achieves a therapeutic dose of a specific component, such as platelets, and maximizes the amount of pooled doses available.

### Summary

There are several aspects of the present subject matter which may be embodied separately or together in the devices and systems described and claimed below. These aspects may be employed alone or in combination with other aspects of the subject matter described herein, and the description of these aspects together is not intended to preclude the use of these aspects separately or the claiming of such aspects separately or in different combinations as set forth in the claims appended hereto.

In one aspect, a method is provided for creating a plurality of fluid products each having a minimum content of a fluid component. The method includes providing a plurality of intermediate fluid volumes each having a known content of a fluid component, determining a plurality of combinations of at least two of the intermediate fluid volumes and pooling the plurality of intermediate fluid volumes according to the determined plurality of combinations so as to create a plurality of fluid products each having a content of the fluid component at least equal to the minimum content of the fluid component. When determining how to combine the intermediate fluid volumes, each intermediate fluid volume is assigned to only one of the combinations, with each combination having a content of the fluid component at least equal to a minimum content of the fluid component for a fluid product. The intermediate fluid volumes are assigned for each combination so as to maximize the number of combinations each having a content of the fluid component at least equal to the minimum content of the fluid component for the fluid product.

In another aspect, a fluid component pooling system is provided. The fluid component pooling system is for use in combination with a fluid flow circuit including a plurality of source containers each holding an intermediate fluid volume with a known content of a fluid component and a plurality of fluid product containers for holding a fluid product with a minimum content of the fluid component. The system includes a pump, a clamp, and a controller coupled to the pump and the clamp. The controller is configured to actuate the pump and the clamp to create a plurality of fluid products each having a minimum content of the fluid component. The controller is configured to determine a plurality of combinations of at least two of the intermediate fluid volumes in which each intermediate fluid volume is assigned to only one of said plurality of combinations, each combination has a content of the fluid component at least equal to the minimum content of the fluid component for the fluid product, and said at least two of the intermediate fluid volumes are assigned for each combination so as to maximize the number of combinations each having a content of the fluid component at least equal to the minimum content of the fluid component for the fluid product. The controller actuates the pump and the clamp to pool the intermediate fluid volumes into the fluid product containers according to the determined plurality of combinations, with each combination of pooled intermediate fluid volumes defining a fluid product having a content of the fluid component at least equal to the minimum content of the fluid component.

These and other aspects of the present subject matter are set forth in the following detailed description of the accompanying drawings.

### Brief Description of the Drawings

Fig. 1 is a flow chart illustrating a general approach to optimization of a pooling process, according to an aspect of the present disclosure;
Fig. 2 is an example of one hundred intermediate products being assigned to "bins" according to the amount of a fluid component present in each intermediate product;
Fig. 3 is an example of how the approach of Fig. 1 may be applied to combining the one hundred intermediate products of Fig. 2 so as to maximize the number of final products each having at least a target content of the fluid component;
Figs. 4A and 4B are portions of a flowchart more particularly illustrating the steps implemented in an exemplary pooling process carrying out the general approach of Fig. 1; and
Fig. 5 is a schematic view of a fluid component pooling system according to an aspect of the present disclosure.

### Description of the Illustrated Embodiments

The embodiments disclosed herein are for the purpose of providing a description of the present subject matter, and it is understood that the subject matter may be embodied in various other forms and combinations not shown in detail. Therefore, specific designs and features disclosed herein are not to be interpreted as limiting the subject matter as defined in the accompanying claims.

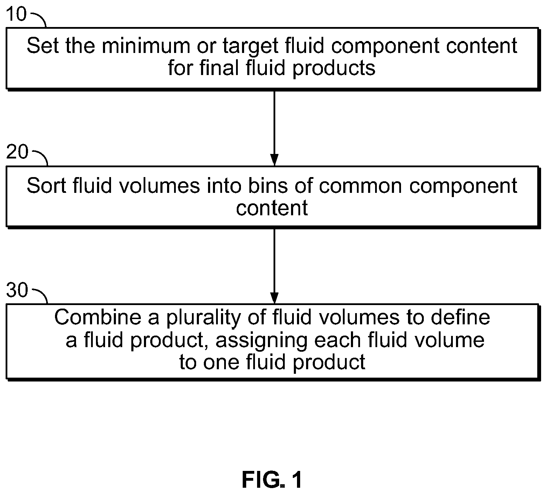

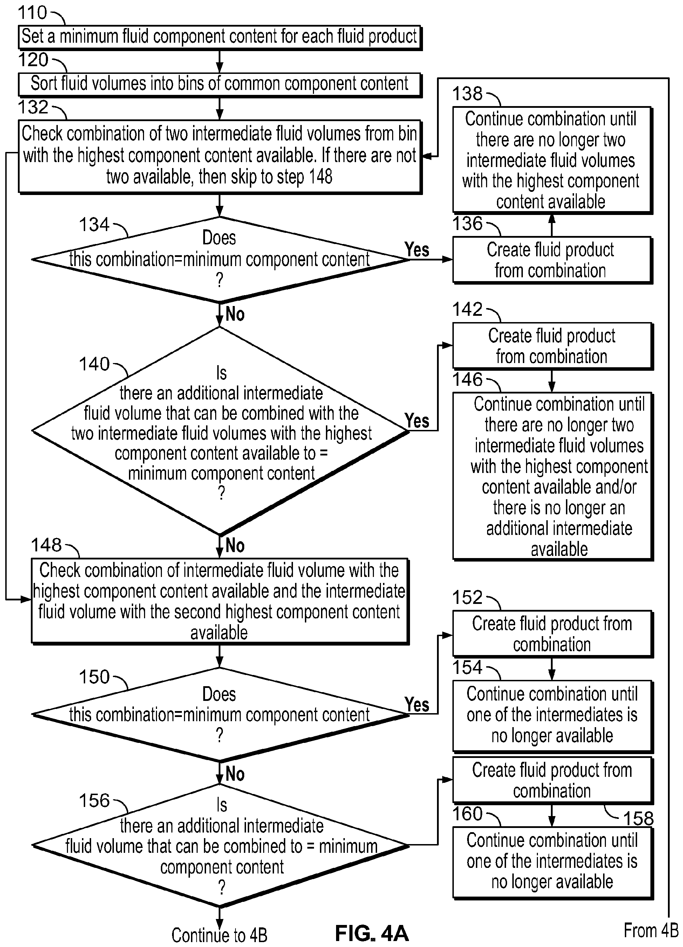

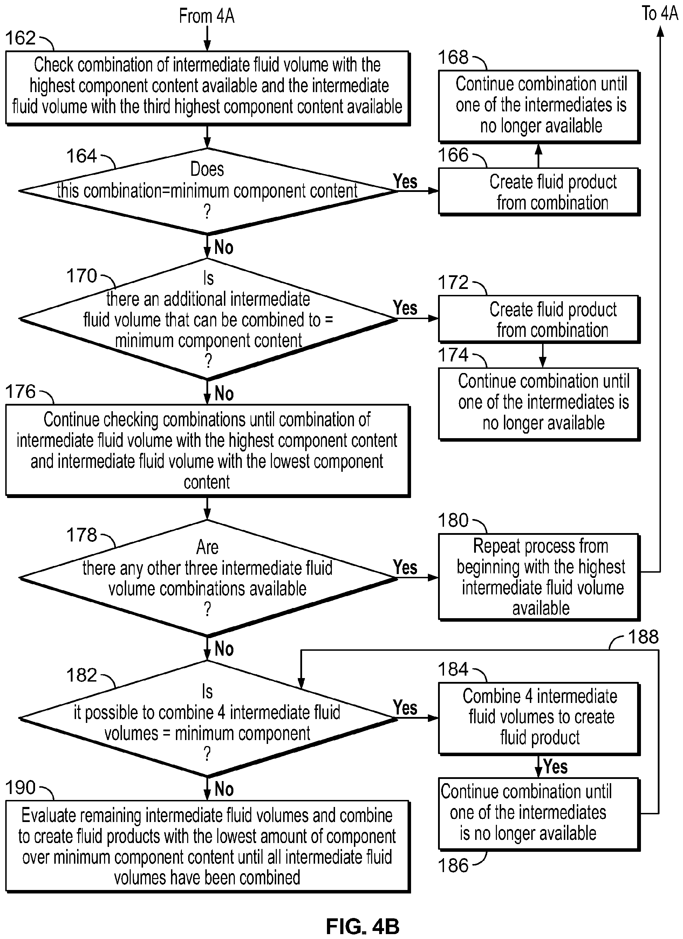

Disclosed herein are computer-based methods for optimizing pooling of volumes of fluid so as to maximize the number of final fluid products with at least a minimum amount of a fluid component. Fig. 1 outlines the overall process which includes setting the minimum or target fluid component content for (final) fluid products (step 10), sorting fluid volumes (which may be base or intermediate fluids) into "bins" of common component content and combining a plurality of fluid volumes to define a fluid product (step 20), and assigning each fluid volume to one fluid product so as to maximize the number of fluid products each having at least the minimum fluid component content (step 30). An exemplary implementation of this third step 30 is presented in greater detail in Figs. 4A and 4B (at steps 132-190), with the first and second steps 10 and 20 also being shown (at steps 110 and 120, respectively) in Fig. 4A.

Techniques according to the present disclosure will be explained in the context of pooling a plurality of intermediate platelet volumes (e.g., volumes of buffy coats or platelet rich plasma or platelet concentrate) so as to form final platelet products, but it should be understood that the techniques described herein are not limited to the pooling of volumes of platelets, but may be applied to other fluids and other fluid components. For example, the component can be any component desirable for a particular treatment, including, but not limited to, components such as platelets, red or white blood cells, T-cells, stems cells, etc. Typically, these cells are pulled or separated from a base starting fluid such as whole blood, with a unit or other amount of whole blood being processed to produce a single intermediate fluid volume having some amount of the cells. Any of a number of possible approaches may be applied to separate the base fluid, including (but not limited to) centrifugation, separation via a spinning membrane, and filtration.

The amount of the component in the intermediate fluid volumes can be measured by any known method. In an exemplary embodiment, the amount of component is measured by optical detection. In other exemplary embodiments, the amount of component is measured as part of complete blood count, by flow cytometry, or any other cell quantification method completed by an automated cell counting system.

Cell component therapies typically require a minimum amount of a specific component to perform the process, such as a blood transfusion. For example, in the case of platelets, the desired therapeutic amount is typically 3.0e11, but it should be understood that the target or minimum content of a fluid component in a final fluid product may vary without departing from the scope of the present disclosure.

In some cases, the amount of cells yielded from the unit or other amount of starting fluid will not be sufficient for a therapeutic dose of the cells, in which case a plurality of intermediate volumes of the separated component (e.g., buffy coat or platelet rich plasma or platelet concentrate, when a platelet product is to be produced) are combined or pooled to produce a final product having at least the target or minimum cell content. The exact manner in which the intermediate fluid volumes are combined or pooled to produce the final product may vary without departing from the scope of the present disclosure.

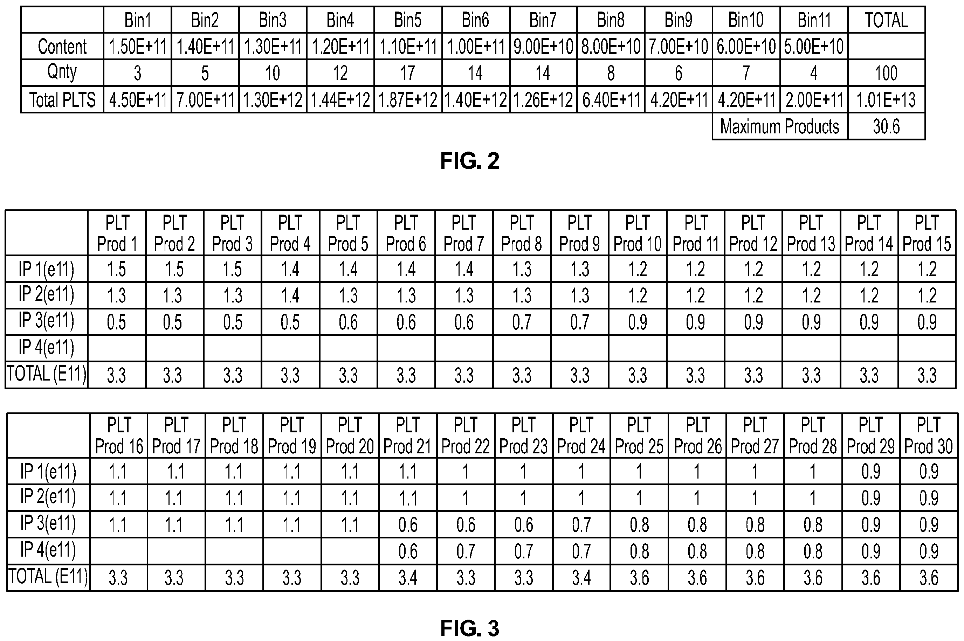

When the content of the fluid component in each of a plurality of intermediate fluid volumes is known, the intermediate fluid volumes can be assigned to sorting bins with other intermediate fluid volumes that have the same content (step 20 of Fig. 1 and step 120 of Fig. 4A). Fig. 2 shows an example of one hundred intermediate fluid volumes that are separated into bins based on the platelet content of the intermediate fluid volume. Although eleven bins are shown, more or fewer bins or containers may be provided depending on the distribution of component contents of the intermediate fluid volumes. In the example, the row labeled "Content" indicates the amount of platelets in the intermediate fluid volumes of that bin or container. The row labeled "Qnty" is the number of intermediate fluid volumes that each have that particular content and are present in the bin or container. The "Total PLTS" row shows the total amount of platelets in each container or bin (i.e., the number in the "Content" row multiplied by the number in the "Qnty" row), with the last column of each of the "Qnty" and "Total PLTS" rows showing the total number of intermediate fluid volumes and the total number of platelets, respectively.

The bottom row of the last column of Fig. 2 presents the maximum number of (final) platelet products having a platelet content of 3.3e11 platelets that may be produced based on the total number of platelets available in the one hundred intermediate fluid volumes. In the illustrated example, 30.6 final platelet products or therapeutic doses of platelets (each having 3.3e11 platelets) may be theoretically produced using the available fluid volumes. As explained above, a conventional approach to platelet pooling is to combine four intermediate volumes, in which case twenty-five final platelet products could be produced using one hundred intermediate volumes.

The number 3.3e11 is used in the illustrated example instead of 3.0e11 (which is a typical platelet content for a therapeutic dose) for various reasons. For example, it may be desirable to select a number greater than the minimum number in order to better ensure that the minimum platelet content is achieved. This may be prudent on account of the expected efficiency of the particular procedure employed to pool each combination of intermediate fluid volumes and subsequent post-processing, such as separating the platelet product, and performing other post-processing procedures so that the final platelet product is a therapy ready product. For example, if the pooling and post-processing processes have an estimated or calculated efficiency of 90%, an intermediate fluid volume having 1.1 e11 platelets can be expected to contribute approximately 1.0e11 platelets to a final therapy ready product. In this case, a combination of intermediate fluid volumes having a total of 3.3e11 platelets may be expected to result in a final product actually having 3.0x11 platelets. As the number can clearly vary, the first step (identified in Fig. 1 at 10 and in Fig. 4A at 110) in effectively pooling the intermediate fluid volumes is setting a minimum component content for each (final) fluid product.

After the intermediate fluid volumes (which may be collected and stored according to any suitable approach without departing from the scope of the present disclosure) have been sorted by their content of the fluid component of interest (as in step 120 of Fig. 1 and step 120 of Fig. 4A), the next step of the algorithm (step 30 of Fig. 1) is applied to assign the intermediate fluid volumes into combinations to be pooled to produce the maximum number of final platelet products, which is detailed in a series of steps starting with 132 in Fig. 4A. Fig. 3 shows an end result of the algorithm being applied to the one hundred intermediate fluid volumes of Fig. 2 to produce thirty final platelet products each having at least 3.3e11 platelets, while Figs. 4A and 4B show the general steps of an algorithm implemented by a computer or other device in distributing the fluid volumes of Fig. 2 into the combinations shown in Fig. 3. It will be seen that the end result shown in Fig. 3 (with thirty combinations) is very close to the theoretical maximum number of final platelet products (30.6) and much greater than the number of platelet products produced when employing a conventional approach (twenty-five).

A plurality of combinations of at least two intermediate fluid volumes are determined, with the most efficient combinations of the intermediate fluid volumes being determined through a series of steps. Before assigning any particular intermediate fluid volume to a combination, the availability of complementary intermediate fluid volumes is assessed. The bin with the intermediate fluid volumes each having the largest content of the fluid component (in the illustrated example, Bin1) is considered first (step 132 of Fig. 4A). After determining the largest content of the fluid component available, the possible combinations are assessed. This assessment is done by first evaluating if an intermediate fluid volume having the largest content of the fluid component can be added to another intermediate volume to meet the minimum component value in the final fluid product (step 134 of Fig. 4A). For instance, if two or more intermediate fluid volume having 1.65e11 platelets are available, they could be combined to meet the minimum content (of 3.3e11 platelets) and may be assigned to a first combination, as in step 136 of Fig. 4A. Depending on the supply of intermediate fluid volumes having such a fluid component content, multiple combinations may be created, as in step 138 of Fig. 4A. When the combinations in step 138 are exhausted, the algorithm will continue from step 148 and proceed through the algorithm for the remaining intermediate volumes.

It should be understood that step 134 of Fig. 4A is specific to a combination of two intermediate fluid volumes with a combined fluid component content equal to the minimum or target content for the final fluid product. Stated differently, the general approach is to first create combinations each using the fewest possible number of intermediate fluid volumes so as to arrive at a combined content of the fluid component equal to the minimum or target content. For example, if multiple intermediate fluid volumes each having 1.7e11 platelets are available, two of these intermediate fluid volumes could be combined to meet the minimum content (of 3.3e11 platelets). However, this may not be the most efficient approach since it would produce a final product having a platelet content greater than the minimum or target content. Thus, in this example, the result of the assessment performed in step 134 of Fig. 4A would be "NO" and the computer or other device implementing the algorithm would advance to step 140.

If there is only one intermediate fluid volume in the bin of those intermediate fluid volumes having the greatest fluid component content, the computer or other device implementing the algorithm advances to step 148 of Fig. 4A. In steps 148 and 150, a combination of the one intermediate fluid volume having the greatest fluid component content and one of the intermediate fluid volumes having the second-highest fluid component content is assessed to determine whether the two fluid volumes have a combined fluid component content equal to the minimum or target content of the final fluid product. For example, if the one intermediate fluid volume with the greatest platelet content has 1.7e11 platelets and an intermediate fluid volume with the second-highest platelet content has 1.6e11 platelets, they could be combined to meet the minimum content (of 3.3e11 platelets) and may be assigned to a combination, as in step 152 of Fig. 4A.

The algorithm may also advance to step 148 of Fig. 4A if there are multiple intermediate fluid volumes each having the greatest fluid component content when the combination of two of those volumes exceeds the target or minimum content of the final fluid product (e.g., if the intermediate fluid volumes with the greatest platelet content each have 1.7e11 platelets and the target or minimum content is 3.3e11 platelets). In this case, if the combination of one of the intermediate fluid volumes with the greatest fluid component content and one of the intermediate fluid volumes with the second-highest fluid component content results would result in a combined fluid component content equal to the minimum or target content (i.e., if the result of the assessment performed in step 150 of Fig. 4A is "YES"), they could be assigned to a combination, as in step 152 of Fig. 4A. Depending on the supply of such intermediate fluid volumes, multiple combinations may be created, as in step 154 of Fig. 4A. When these combinations are no longer possible, the algorithm advances to step 162 with the remaining intermediate volumes (if any) and continues the process.

This approach of assessing the combination of the intermediate fluid volume having the greatest fluid component content and one other intermediate fluid volume may continue, with the first intermediate fluid volume being compared to a second intermediate fluid volume having successively lower fluid component contents. For example, after assessing the combination of two intermediate fluid volumes having the greatest fluid component content (steps 132 and 134 of Fig. 4A) and the combination of one intermediate fluid volume having the greatest fluid component content and one intermediate fluid volume having the second-highest fluid component content (steps 148 and 150 of Fig. 4A), the combination of one intermediate fluid volume having the greatest fluid component content and one intermediate fluid volume having the third-highest fluid component content may be assessed (steps 162 and 164 of Fig. 4B). Once this combination has been assessed (and either accepted (advancing the algorithm to steps 166 and 168 of Fig. 4B until the combinations are exhausted and the algorithm moves to step 176) or rejected), an intermediate fluid volume having the next highest fluid component content is assessed and the process is repeated until the two-volume combination of one intermediate fluid volume from each bin and one intermediate fluid volume having the greatest fluid component content has been assessed (step 176 of Fig. 4B). Once again, it is stressed that a particular intermediate fluid volume is not available for assessment as part of a combination once it has been assigned to some other combination.

In the example shown in the spreadsheet of Fig. 2, Bin1 includes three intermediate fluid volumes each having 1.5e11 platelets. Two of these could be combined to produce a final product having 3.0e11 platelets, which is less than the minimum or target content of 3.3e11 platelets (i.e., the result of the assessment performed in step 134 of Fig. 4A is "NO"). Thus, the computer or device implementing the algorithm would determine that each combination of intermediate fluid volumes must include at least three intermediate fluid volumes in order to achieve the target or minimum platelet content for the final platelet product and forego assessing additional two-volume combinations (e.g., assessing the combination of one intermediate fluid volume from Bin1 and one intermediate fluid volume of Bin2, which would take place in steps 148 and 150 of Fig. 4A).

As such, the computer or device will proceed with the algorithm by assessing whether two intermediate fluid volumes having the greatest fluid component content can be combined with a third intermediate fluid volume to meet the minimum or target content in the final fluid product (step 140 of Fig. 4A). In the example of Fig. 2, the first two intermediate volumes (each containing 1.5e11 platelets) are selected from Bin1, the total platelet content is 3.0e11 platelets, such that an intermediate fluid volume having 3.0e10 would be required to produce a final platelet product having the target or minimum 3.3e11 platelets. If an intermediate fluid volume having the proper platelet content is available (and has not been previously assigned to another combination), then the three intermediate fluid volumes will be grouped together as a first combination to be later pooled to create a first platelet product (step 142 of Fig. 4A). Depending on the supply of such intermediate fluid volumes, multiple combinations may be created, as in step 146 of Fig. 4A. After the combinations are no longer available in 146, the algorithm will proceed to step 148 and continue through the algorithm.

However, in the example provided, there are no intermediate fluid volumes containing 3.0e10 platelets. Instead, the smallest available platelet content is 5.0e10 platelets (Bin11), which would be combined with two fluid volumes from Bin1 to produce a final platelet product having 3.5e11 platelets. Although such a combination meets the minimum platelet content requirement, it exceeds that number (3.5e11 platelets vs 3.3e11 platelets), meaning that a combination including two fluid volumes from Bin1 cannot be used to most efficiently group the intermediate fluid products, as the first goal is creating as many final fluid products with the exact fluid component content (3.3e11 platelets, in the illustrated example) using the smallest number of possible intermediate fluid products (three, in the illustrated example). Therefore, the computer or device will proceed with the algorithm by moving on to other possible combinations to create a first fluid product.

The next step is assessing the combination of an intermediate fluid volume having the highest platelet content (1.5e11 platelets from Bin1, in the illustrated example) with an intermediate fluid volume having the second highest platelet content (1.4e11 platelets from Bin2, in the illustrated example) and some third intermediate fluid volume, as shown in Fig. 4A at step 156. Combining 1.4e11 platelets and 1.5e11 platelets creates a combination having a total of 2.9e11 platelets, requiring 4.0e10 platelets to produce a final platelet product having 3.3e11 platelets. If there were an intermediate fluid volume having 4.0e10 platelets, it could be assigned to a combination with the other two intermediate fluid volumes (step 158 of Fig. 4A), with that combination being repeated (step 160 of Fig. 4A), as the supply of available intermediate fluid volumes (i.e., those that have not yet been assigned to some other combination) allows, moving to step 162 when the combination is no longer possible. However, as was the case in the previous combination of three intermediate fluid volumes, an intermediate fluid volume having fewer than 5.0e10 platelets is not available (as shown in Fig. 2). As such, a combination including one intermediate fluid volume from Bin1 and one intermediate fluid volume from Bin2 is rejected (similar to the combination of two intermediate fluid volumes from Bin1 was rejected).

The next step will be again to retain a first intermediate fluid volume having the greatest platelet content (1.5e11 platelets, in the illustrated embodiment) and assess a combination including a second intermediate fluid volume from the bin having the greatest platelet content that has not yet been considered. In the illustrated example, an intermediate fluid volume from Bin1 and from Bin2 have been assessed (and rejected) as the second intermediate fluid volume in a combination, so an intermediate volume from Bin3 (containing 1.3e11 platelets) will be considered as the second intermediate fluid volume in a combination with a first intermediate fluid volume from Bin1 (step 170 of Fig. 4B). Such a combination results in a total of 2.8e11 platelets, with the remaining amount to reach the minimum platelet content in the final fluid product, therefore, being 5.0e10. For the example provided, four intermediate fluid volumes each having 5.0e10 platelets are available in Bin11, so the combination of one intermediate fluid product having 1.5e11 platelets (from Bin1), one intermediate fluid product having 1.3e11 platelets (from Bin3), and one intermediate fluid product having 5.0e10 platelets (from Bin11) can be made to create the first fluid product (identified as PLTProd1 in Fig. 3), as shown in Fig. 4B as step 172. When a successful combination like this is achieved, it can be repeated as available (step 174 of Fig. 4B) until the combination is no longer possible, at which point the algorithm will proceed to step 176 with any remaining intermediate volumes. As shown in the example pooling of Fig. 3, this combination can be made three times (resulting in PLTProd1, PLTProd2, and PLTProd3), with the availability of a 1.5e11 platelet intermediate fluid volume being a limiting factor (because Bin1, with 1.5e11 platelet volumes, has only three intermediate fluid products with that amount).

The computer or other device implementing the algorithm will go through this process with the remaining intermediate fluid volumes that have not yet been assigned to a combination (steps 178 and 180 of Fig. 4B) until there are no other possible combinations of three intermediate products to exactly meet the minimum component content requirement. In the illustrated example, seventeen more combinations of three intermediate fluid volumes each resulting in a final product having the exact target or minimum platelet content (identified in Fig. 3 as PLTProd4 - PLTProd20) may be produced. As shown in Fig. 3, once the supply of intermediate fluid products in Bin1 is exhausted (PLTProd1 - PLTProd3), combinations using the intermediate fluid products having the next highest platelet content (from Bin2) as first intermediate fluid volumes (presented in row "IP 1 (e11) of Fig. 3) are assessed, resulting in four more combinations (PLTProd4 - PLTProd7). Combinations using the intermediate fluid products having the third highest platelet content (1.3e11 from Bin3) as first intermediate fluid volumes are next assessed when the supply of Bin2 has been exhausted, resulting in two more combinations (PLTProd8 and PLTProd9). Finally, combinations using the intermediate fluid products having the fourth highest platelet content (1.2e11 from Bin4) as first intermediate fluid volumes are assessed (resulting in PLTProd10 - PLTProd15), followed by combinations using the intermediate fluid products having the fifth highest platelet content (1.1e11 from Bin5) are assessed (resulting in PLTProd16 - PLTProd20).

When the computer or other device assesses a twenty-first combination (which would be PLTProd21 in Fig. 3), it will find only two remaining intermediate fluid volumes from Bin5 (each having 1.1e11 platelets), such that it is no longer possible to create a combination of three intermediate fluid volumes having exactly 3.3e11 platelets. At that time, the computer or other device will then settle for combinations of four intermediate fluid products to reach the minimum platelet content (steps 182 - 188 of Fig. 4B) or to just exceed the minimum platelet content (e.g., a platelet content of 3.4e11 platelets or 3.5e11 platelets), as in step 190. Using the above-described general approach, the algorithm will be implemented to first create a combination of two intermediate fluid volumes from Bin5 (each having 1.1e11 platelets) and two intermediate fluid volumes from Bin10 (each having 6.0e10 platelets). Such a combination (PLTProd21 in Fig. 3) will result in a final product having a total of 3.4e11 platelets, but a combination having exactly 3.3e11 platelets is no longer possible with two intermediate fluid volumes each having 1.1e11 platelets due to the supply of intermediate fluid volumes each having 5.0e10 platelets (from Bin11) being previously exhausted.

The establishment of combination PLTProd21 exhausts the supply of Bin5, so the computer or other device proceeds to implement the algorithm using intermediate fluid volumes having the next highest available platelet content (of 1.0e11 from Bin6) as first and second components of subsequent combinations. This results in seven more acceptable combinations (PLTProd22 - PLTProd28) before the supply of Bin6 (along with the supplies of Bin8, Bin9, and Bin10) being exhausted. At that point, the only bin having unassigned intermediate fluid volumes is Bin7 (which still has eight intermediate fluid volumes each having 9e10 platelets that have not yet been assigned to a previous combination). These eight intermediate fluid volumes of Bin7 are assigned to two combinations in groups of four (PLTProd29 and PLTProd30 of Fig. 3), with each combination having a total of 3.6e11 platelets. As noted above, it will be seen in Fig. 3 that implementation of the algorithm results in thirty combinations that may each be pooled to create a final product having at least 3.3e11 platelets, which is very close to the theoretical maximum of 30.6 products (Fig. 2).

As noted above, an algorithm according to the present disclosure may be implemented by a computer or other suitable device. Similarly, the configuration of a system for implementing the pooling of the combinations of intermediate fluid volumes assigned by execution of the algorithm may vary without departing from the scope of the present disclosure.

One possible system that can incorporate the algorithm is described in US Patent Publication No. 11,135,343, which is hereby incorporated herein by reference.

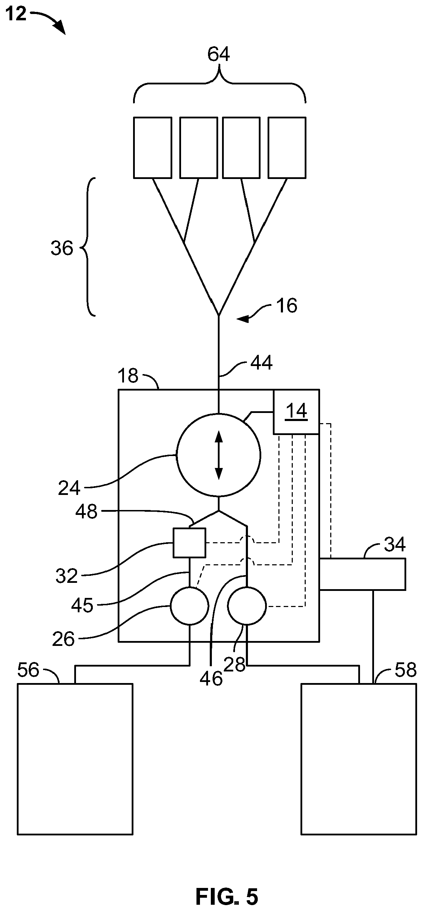

An embodiment of this fluid component pooling system is indicated in general at 12 in Fig. 5. The fluid component pooling system pools the intermediate fluid volumes from a plurality of source containers 64 to a plurality of fluid product containers 56 (only one is shown in Fig. 5).

The source containers with the intermediate fluid volumes are part of a (typically disposable or single-use) fluid flow set 16. The various components of the fluid flow circuit 16 are connected by conduits, including source tubing lines 36 and tubing line 44, which splits into pool tubing line 45 and was tubing line 46 via y-connector 48. The source tubing lines 36 are connected to the intermediate fluid volumes in a plurality of source containers 64 of intermediate fluid volumes. While four source containers 64 are illustrated, any number of source containers may be provided and pooled.

The system 12 can include various clamps (such as pool tubing clamp 26 and a wash tubing clamp 28) configured to interact with the conduits of a fluid flow circuit 16 mounted to the system 12. As examples only, the tubing clamps 26 and 28 may be solenoid pinch valves, motor-driven rotary pinch valves, linear actuators, stop cocks or any other type of automated clamping or valve device known in the art.

The intermediate fluid volumes are pumped by pump 24 through the fluid flow circuit 16 and into the fluid product containers 56. As an example only, the pump may be a peristaltic pump of the type available from Fenwal, Inc. of Lake Zurich, Illinois, which is an affiliate of Fresenius Kabi AG of Bad Homburg, Germany. The single, two-way pump 24 may of course be replaced by two one-way pumps. A wash media container 58 may also be a component of the fluid flow circuit 16 in order to rinse and recover additional cells from the source containers 64. The source containers and fluid product containers may be bags, but can also be any known and functional storage container.

As illustrated in Fig. 5, the device includes a controller 14 which is in electrical communication with the pump 24, and clamps 26 and 28. The controller operates the pump and clamps to move the intermediate fluid volumes from the source containers to the fluid product containers 56. The controller pools selected source containers based on the above-described algorithm.

Components of the system may be included in a housing, indicated at 18, that may be mounted on a pole, set on a surface, or otherwise positioned or mounted so as to be convenient for a user to operate.

The blood component pooling device may also include an optional air detector 32 and an optional weight scale 34. In an embodiment that omits either component, or both components, the operation of the pump may be based on predetermined times based on pump flow rates and/or source blood component and wash media bag volumes.

Although one example of a fluid component pooling system is described, obvious variations to the system, and any other operating pooling system may also utilize the disclosed algorithm to pool the intermediate fluid volumes to the fluid product containers.

### Aspects

Aspect 1. A method for creating a plurality of fluid products each having a minimum content of a fluid component, comprising: providing a plurality of intermediate fluid volumes each having a known content of a fluid component; determining a plurality of combinations of at least two of the intermediate fluid volumes, wherein each intermediate fluid volume is assigned to only one of said plurality of combinations, each combination has a content of the fluid component at least equal to a minimum content of the fluid component for a fluid product, and said at least two of the intermediate fluid volumes are assigned for each combination so as to maximize the number of combinations each having a content of the fluid component at least equal to the minimum content of the fluid component for the fluid product; and pooling the plurality of intermediate fluid volumes according to the determined plurality of combinations, with each combination of pooled intermediate fluid volumes defining a fluid product having a content of the fluid component at least equal to the minimum content of the fluid component.

Aspect 2. The method of Aspect 1, wherein the content of at least one of said plurality of intermediate fluid volumes is determined by optical detection.

Aspect 3. The method of any one of the preceding Aspects, wherein said fluid component comprises platelets.

Aspect 4. The method of Aspect 3, wherein the plurality of intermediate fluid volumes includes at least one volume of buffy coat.

Aspect 5. The method of any one of Aspects 3-4, wherein the plurality of intermediate fluid volumes includes at least one volume of platelet rich plasma.

Aspect 6. The method of any one of Aspects 3-5, wherein the plurality of intermediate fluid volumes includes at least one volume of platelet concentrate.

Aspect 7. The method of any one of the preceding Aspects, wherein said determining the plurality of combinations includes calculating the content of the fluid component of the fluid product defined by each combination based at least in part on an efficiency at which each combination is pooled to produce the fluid product.

Aspect 8. The method of any one of the preceding Aspects, further comprising establishing a plurality sorting bins, and assigning each intermediate fluid volume to one of said sorting bins, with each sorting bin corresponding to a different fluid component content.

Aspect 9. The method of any one of the preceding Aspects, wherein determining a first combination includes assigning to the first combination the intermediate fluid volume having the greatest content of the fluid component.

Aspect 10. The method of any one of the preceding Aspects, wherein determining each combination includes assigning to the combination the intermediate fluid volume having the greatest content of the fluid component among the intermediate fluid volumes not previously assigned to another combination.

Aspect 11. The method of any of the preceding Aspects, wherein determining each combination includes: assigning to the combination the two intermediate fluid volumes having the greatest content of the fluid component among the intermediate fluid volumes not previously assigned to another combination, determining a difference between the minimum content of the fluid component and the combined content of the fluid component of said two intermediate fluid volumes, determining whether there is an intermediate fluid volume that has a content of the fluid component equal to said difference and that has not been previously assigned to another combination, assigning to the combination said intermediate fluid volume having a content of the fluid component equal to said difference when such an intermediate fluid volume is available, and rejecting the combination when no such intermediate fluid volume is available.

Aspect 12. The method of any one of the preceding Aspects, wherein determining the plurality of combinations includes assigning the intermediate fluid volumes so as to produce combinations of a number of intermediate fluid volumes each having the minimum content of the fluid component before assigning a greater number of intermediate fluid volumes to a combination so as to produce a combination exceeding the minimum content of the fluid component.

Aspect 13. The method of any one of the preceding Aspects, wherein determining the plurality of combinations includes: assigning the intermediate fluid volumes so as to produce combinations of three intermediate fluid volumes each having the minimum content of the fluid component, when it is no longer possible to produce a combination of three intermediate fluid volumes having the minimum content of the fluid component, assigning the intermediate fluid volumes so as to produce subsequent combinations of four intermediate fluid volumes each having the minimum content of the fluid component, and when it is no longer possible to produce a combination of four intermediate fluid volumes having the minimum content of the fluid component, assigning to each subsequent combination the intermediate fluid volumes having the lowest content of the fluid component until producing a combination having at least the minimum content of the fluid component.

Aspect 14. A fluid component pooling system for use in combination with a fluid flow circuit including a plurality of source containers each holding an intermediate fluid volume with a known content of a fluid component and a plurality of fluid product containers for holding a fluid product with a minimum content of the fluid component, the system comprising: a pump; a clamp; and a controller coupled to the pump and the clamp and configured to actuate the pump and the clamp to create a plurality of fluid products each having a minimum content of the fluid component, wherein the controller is configured to determine a plurality of combinations of at least two of the intermediate fluid volumes in which each intermediate fluid volume is assigned to only one of said plurality of combinations, each combination has a content of the fluid component at least equal to the minimum content of the fluid component for the fluid product, and said at least two of the intermediate fluid volumes are assigned for each combination so as to maximize the number of combinations each having a content of the fluid component at least equal to the minimum content of the fluid component for the fluid product, and actuate the pump and the clamp to pool the intermediate fluid volumes into the fluid product containers according to the determined plurality of combinations, with each combination of pooled intermediate fluid volumes defining a fluid product having a content of the fluid component at least equal to the minimum content of the fluid component.

Aspect 15. The system of Aspect 14, wherein said fluid component comprises platelets.

Aspect 16. The system of Aspect 15, wherein the plurality of intermediate fluid volumes includes at least one of volume of buffy coat.

Aspect 17. The system of any one of Aspects 15-16, wherein the plurality of intermediate fluid volumes includes at least one volume of platelet rich plasma.

Aspect 18. The system of any one of Aspects 15-17, wherein the plurality of intermediate fluid volumes includes at least one volume of platelet concentrate.

Aspect 19. The system of any one of Aspects 14-18, further comprising a second clamp coupled to the controller, wherein the controller is configured to actuate the pump and the second clamp to convey a wash medium through the fluid flow circuit.

Aspect 20. The system of any one of Aspects 14-19, further comprising an air detector.

It will be understood that the embodiments and examples described above are illustrative of some of the applications of the principles of the present subject matter. Numerous modifications may be made by those skilled in the art without departing from the spirit and scope of the claimed subject matter, including those combinations of features that are individually disclosed or claimed herein. For these reasons, the scope hereof is not limited to the above description but is as set forth in the following claims, and it is understood that claims may be directed to the features hereof, including as combinations of features that are individually disclosed or claimed herein.

## Claims

1. A method for creating a plurality of fluid products each having a minimum content of a fluid component, comprising:
providing a plurality of intermediate fluid volumes each having a known content of a fluid component;
determining a plurality of combinations of at least two of the intermediate fluid volumes, wherein
each intermediate fluid volume is assigned to only one of said plurality of combinations,
each combination has a content of the fluid component at least equal to a minimum content of the fluid component for a fluid product, and
said at least two of the intermediate fluid volumes are assigned for each combination so as to maximize the number of combinations each having a content of the fluid component at least equal to the minimum content of the fluid component for the fluid product; and
pooling the plurality of intermediate fluid volumes according to the determined plurality of combinations, with each combination of pooled intermediate fluid volumes defining a fluid product having a content of the fluid component at least equal to the minimum content of the fluid component.

2. The method of claim 1, wherein the content of at least one of said plurality of intermediate fluid volumes is determined by optical detection.

3. The method of any one of the preceding claims, wherein said fluid component comprises platelets.

4. The method of claim 3, wherein the plurality of intermediate fluid volumes includes at least one of volume of buffy coat, at least one volume of platelet-rich plasma, and/or at least one volume of platelet concentrate.

5. The method of any one of the preceding claims, wherein said determining the plurality of combinations includes calculating the content of the fluid component of the fluid product defined by each combination based at least in part on an efficiency at which each combination is pooled to produce the fluid product.

6. The method of any one of the preceding claims, further comprising establishing a plurality of sorting bins, and
assigning each intermediate fluid volume to one of said sorting bins, with each sorting bin corresponding to a different fluid component content.

7. The method of any one of the preceding claims, wherein determining a first combination includes assigning to the first combination the intermediate fluid volume having the greatest content of the fluid component.

8. The method of any one of the preceding claims, wherein determining each combination includes assigning to the combination the intermediate fluid volume having the greatest content of the fluid component among the intermediate fluid volumes not previously assigned to another combination.

9. The method of any one of the preceding claims, wherein determining each combination includes:
assigning to the combination the two intermediate fluid volumes having the greatest content of the fluid component among the intermediate fluid volumes not previously assigned to another combination,
determining a difference between the minimum content of the fluid component and the combined content of the fluid component of said two intermediate fluid volumes,
determining whether there is an intermediate fluid volume that has a content of the fluid component equal to said difference and that has not been previously assigned to another combination,
assigning to the combination said intermediate fluid volume having a content of the fluid component equal to said difference when such an intermediate fluid volume is available, and
rejecting the combination when no such intermediate fluid volume is available.

10. The method of any one of the preceding claims, wherein determining the plurality of combinations includes assigning the intermediate fluid volumes so as to produce combinations of a number of intermediate fluid volumes each having the minimum content of the fluid component before assigning a greater number of intermediate fluid volumes to a combination so as to produce a combination exceeding the minimum content of the fluid component.

11. The method of any of the preceding claims, wherein determining the plurality of combinations includes:
assigning the intermediate fluid volumes so as to produce combinations of three intermediate fluid volumes each having the minimum content of the fluid component,
when it is no longer possible to produce a combination of three intermediate fluid volumes having the minimum content of the fluid component, assigning the intermediate fluid volumes so as to produce subsequent combinations of four intermediate fluid volumes each having the minimum content of the fluid component, and
when it is no longer possible to produce a combination of four intermediate fluid volumes having the minimum content of the fluid component, assigning to each subsequent combination the intermediate fluid volumes having the lowest content of the fluid component until producing a combination having at least the minimum content of the fluid component.

12. A fluid component pooling system for use in combination with a fluid flow circuit including a plurality of source containers each holding an intermediate fluid volume with a known content of a fluid component and a plurality of fluid product containers for holding a fluid product with a minimum content of the fluid component, the system comprising:
a pump;
a clamp; and
a controller coupled to the pump and the clamp and configured to actuate the pump and the clamp to create a plurality of fluid products each having a minimum content of the fluid component, wherein the controller is configured to
determine a plurality of combinations of at least two of the intermediate fluid volumes in which each intermediate fluid volume is assigned to only one of said plurality of combinations, each combination has a content of the fluid component at least equal to the minimum content of the fluid component for the fluid product, and said at least two of the intermediate fluid volumes are assigned for each combination so as to maximize the number of combinations each having a content of the fluid component at least equal to the minimum content of the fluid component for the fluid product, and
actuate the pump and the clamp to pool the intermediate fluid volumes into the fluid product containers according to the determined plurality of combinations, with each combination of pooled intermediate fluid volumes defining a fluid product having a content of the fluid component at least equal to the minimum content of the fluid component.

13. The system of claim 12, wherein
said fluid component comprises platelets, and
the plurality of intermediate fluid volumes includes at least one of volume of buffy coat, at least one volume of platelet rich plasma, and/or at least one volume of platelet concentrate.

14. The system of any one of claims 12-13, further comprising a second clamp coupled to the controller, wherein the controller is configured to actuate the pump and the second clamp to convey a wash medium through the fluid flow circuit.

15. The system of any one of claims 12-14, further comprising an air detector.
